# EUROPEAN PATENT APPLICATION

(11) **EP 1 862 155 A1**
(43) Date of publication of application: **05.12.2007**
(21) Application number: 06114641.1
(22) Date of filing: 29.05.2006
(51) Int. Cl.: A61F 13/15

(54) **Absorbent pad**

(71) Applicant: PAPER-PAK SWEDEN AB, 578 24 Aneby (SE)
(72) Inventor: Bruun, Jonas, SE-222 21, Lund (SE); Lindqvist, Arne, SE-151 35, Södertälje (SE); Hallgren, Sammy, SE-212 19, Malmö (SE)
(74) Representative: Kraenzmer, Martin

(57) **Abstract**

According to the invention it is disclosed an absorbent pad (1) comprising a liquid-impervious back sheet (2), a plurality of bodies (3) of liquid-absorbent material disposed on one surface of the back sheet (2), and a liquid-pervious cover sheet (4) covering said bodies (3) of absorbent material and secured to the back sheet (2) along channels (7) which separate said bodies (3) of absorbent material. The channels (7) progresses in a channel-pattern in which three channels (7), meeting in a joint, form respective edges (6) of said whole bodies (3) and said channels (7) being substantially free from absorbent material. A method for the manufacturing of such absorbent pad (1) is also disclosed.

## Description

### Field of the Invention

The present invention relates to absorbent pads, more particularly absorbent pads of the type used as incontinence pads to be positioned under a person.

### Background of the Invention

It is known to make absorbent pads with flow-spreading depressions or projections. Sanitary napkins, baby diapers and the like have been made with cellulose fluff or crepe wadding material between a cover sheet and an impervious back sheet, and with the absorbent medium embossed with depressions extending transverse to the longitudinal axis of the pad or in various patterns such as diamond, herringbone, sinusoidal, etc.

An alternative on the constructions discussed above involved the application of lines of glue or other adhesive to the plastic backing sheet to hold the absorbent mat in place. A diamond pattern is shown in US 3, 881,487, wherein an embodiment in which the channels are filled with adhesive i disclosed. By adding adhesive the flow of liquid through the channels may be impeded.

US 4,360,021 discloses an embossed plastic sheet which provides tiny depressions having small quantities of superabsorbent material in the depressions. A cover of non-woven material is disposed over the depressions and the liquid is discharged directly onto the cover and into the depressions. As one depression is filled and the liquid absorbed by the filling material, excess liquid spills over into the next adjacent depression, and continues until the liquid is fully absorbed in the nearest depressions. Such a structure has no channels for flow of the liquid.

It is known from EP 394 274 to provide absorbent pads containing diamond-shaped pockets between channels, such as in fig. 1 of the document in question. However, it is sometimes experienced that such pockets are not filled accurately with fluff during manufacturing. This in turn may reduce its capability of effeciently absorb liquid. There is a risk that small basins are formed at locations, especially near channels and in particular where channels are crossing, where the content of fluff in a pocket is not sufficient. Furthermore, it is also desirable for comfort reasons that each pocket is filled to a sufficient degree in order to provide the targeted cussioning effect. However, the issue of applying pockets containing fluff in a manner that will reduce the risk for nearlyying "basins"has not been adressed in the prior art.

It is accordingly an object of the present invention to provide an absorbent pad, for example of the incontinence pad type, having improved characteristics of liquid flow and absorption. It is of importance for products and articles of the kind that they are experienced as comfortable and reliable by a person during use. Another object of the present invention is the provision of such a pad, whose construction reduces the chance of liquid leakage or spillage. Still another object of the present invention is the provision of such a pad, which will be easy and inexpensive to manufacture and rugged and durable in use.

### Summary of the Invention

It is accordingly an oject of the present invention to provide an absorbent pad, for example of the incontinence pad type having improved characteristics of reduction of chance of leakage or spillage. Furthermore, it is an object to provide an improved absorbent pad having improved characteristics of absorption and at the same time provides for comfort for the person using it. Still another object of the present invnetion is the provision of such a pad, which will be easy and inexpensive to manufacture and rugged and durable in use.

Hence, it is accomplished an absorbent pad according to the invention comprising a liquid-impervious back sheet, a plurality of bodies of liquid-absorbent material disposed on one surface of the back sheet, and a liquid-pervious cover sheet covering said bodies of absorbent material and secured to the back sheet along channels which separate said bodies of absorbent material, characterized in that the channels progresses in a channel-pattern in which three channels, meeting in a joint, form respective edges of said whole bodies and said channels being substantially free from absorbent material.

It is realized that the provision of a pattern in accordance with the characterizing portion of claim 1 provides for channels which allows for keeping the fluff in position and yet providing means for ventilation even after a discharge has been absorbed by the absorbent pad. The bodies also enables improved fluff filling characteristics for each absorbent pad body due to the possible avoidance of sharped angled corners. It is implicit from the wording of the claim that the absorbent bodies has at least 5 edges forming the circumferential edge of a whole body. Hence, the absorbent pad according to the invention provides for improved capability of isolating fluid by absorption in a specific area of the absorbent pad. Furthermore, the stiffness of the absorbent pad may be increased when having joints with three channels meeting instead of for instance long straight channels, which makes the pad easily foldable and possibly easily shrunk dring use leading to lesser comfort experience. The provision of filled bodies also enables a high specific surface of the absorbent pad as a whole. A higher specific surface may be utilized for enhancing the capillary effect for absorption.

Advantageously, the edges of said whole body has an angle of 105-135°, preferably 118-122°, in relation to nearlying edges thereof. A small angle will reduce the possibility to fully fill the body with fluff leading to basins instead of absorbing corners.

Preferably, said cover sheet is adhesively bonded to said back sheet propagating along said channels. Still more preferably, said cover sheet is cohesively secured to said back sheet along said channels. The back sheet is substantially flat and said cover sheet has pockets therein and containing said bodies.

More particularly, said bodies contains 6 edges and hence the whole bodies together forms a honeycomb pattern. The provision of a three channel joint hexagon pattern is a preferred alternative due to the symmetric shape in addition to all the listed advantages. Hence, said three channels propagates evenly distributed, in the plane of said absorbent pad, from said joint.

There is also provided a method of making an absorbent pad from a liquid-impervious back sheet and a liquid-pervious cover sheet and absorbent material between said sheets, comprising forming in the cover sheet a plurality of bodies, filling the bodies with a separate quantity of absorbent material in each body, and securing the back sheet to the cover sheet along channels between the bodies, characterized in that the channels progresses in a channel-pattern in which three channels meet in joints forming respective edges of said whole bodies and said channels being substantially free from absorbent material.

### Brief Description of the Drawings

The above and other objects and features of the present invention will be better understood in view of the following detailed description, taken in connection with the accompanying drawings, in which;
Fig. 1 is a perspective view of a preferred embodiment of the present invention.
Fig. 2 is a top plan view of an absorbent pad according to the present invention and an enlarged image of a channel joint,
Fig. 3 is a cross sectional view thereof on the line III-III of Fig. 2,
Fig. 4 is a perspective view of a second preferred embodiment of the present invention.

### Detailed Description of the Invention

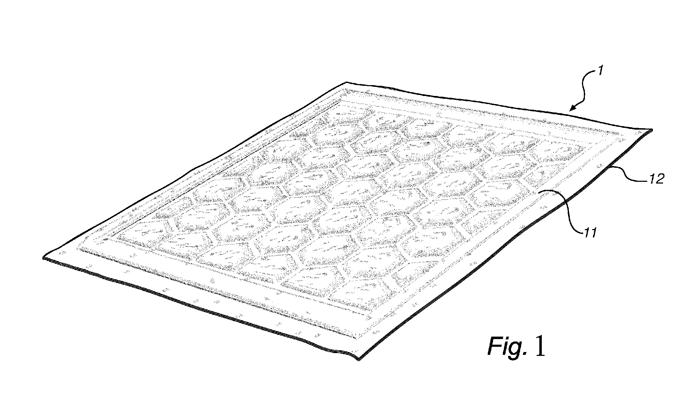

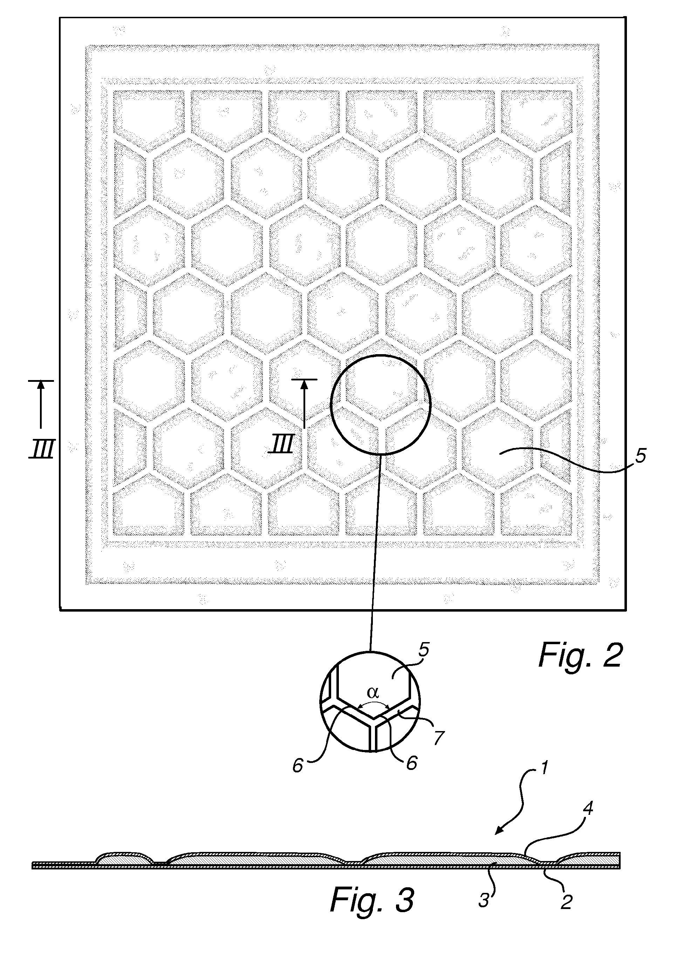

Referring now to the drawings in greater detail, and first to Figs. 1-3 thereof, there is shown an absorbent pad 1 according to the present invention, in the form of an incontinence pad 1 of the type used beneath the body of a human in need of the same. The pad 1 includes a liquid-impervious back sheet 2. The material of sheet 2 can be that which is conventionally used as the liquid-impervious back sheet 2 for diapers and incontinence pads and the like, for example polyethylene film of a thickness of 0.010 mm to 0.050 mm, preferably 0.015 to 0.030 mm.

On top of the sheet 2 are a plurality of spaced bodies 3 of absorbent material, preferably fibrous in nature. This absorbent material is quite conventional and has a high capacity to absorb liquid. A typical such absorbent is a fluff of bleached sulphate pulp made from softwood and having a fiber lengh of 0.1 mm to 10 mm, preferably 2-5 mm. However, any other conventional absorbent could be used.

The bodies 3 are contained in pockets formed in a continuous liquid-pervious cover sheet 4 that overlies the bodies 3 and is directly secured to back sheet 2 along channels 7 between bodies 3. The channels 7 thus define, in the illustrated embodiment a certain pattern. When the bodies 3 are in the form of islands, it is preferred that they have a size from 10 x 10 mm up to 100 x 100 mm. A typical size for the diameter of the body 3 as shown in the drawings, is 70 mm. The channels 7 can vary in width from 0.5 mm up to 10 mm, but are preferably 1-4 mm in width.

The pockets can vary in depth from 1 mm to 20 mm or even more. The cover sheet 4 can be woven or non-woven, preferably non-woven. A typical example of such non-woven material is extruded fibrous polypropylene. Such non-woven fabric is conventional in the art of disposable diapers and the like and has a weight of 8-35 g/m² or more.

However, the invention is not restricted to a non-woven cover sheet. Woven material is also within the contemplation of the invention. In short, the materials of the back sheet 2, the absorbent bodies 3 and the cover sheet 4 can all be conventional. It is their arrangement and relationship to each other that characterize the present invention.

The pad 1 can also be provided with one or more pockets forming a margin filled with absorbent material. Outwardly of margin 11 is a border 12, in which, the cover sheet 4 is directly secured to back sheet 2. This securement may be accmplished in the same manner as the channels 7. The resulting pad 1, if an incontinence pad 1, can be of conventional size and shape for such purpose. Suitably, the pad 1 has a length and width, for example, of about 75 cm and 60 cm.

In use, the pad 1, if an incontinence pad 1, is placed beneath the incontinent person, whose weight is borne by the filled pockets, leaving open the channels 7 between them. Liquid from the person's body readily penetrates the liquid-pervious cover sheet 4 and is absorbed by the immediately subjacent bodies 3 of absorbent material. When these are full, then the excess liquid passes along the channels 7 to the nearest dry pockets, where the liquid is absorbed from the channels 7 through the side walls of those pockets and into the dry absorbent material in those next pockets. This can proceed until substantially all the pad 1 is saturated.

Only thereafter will there be danger of liquid leakage or spillage. In the meantime, however, maximum utilization of the ability of the pad 1 to absorbent liquid, has been achieved.

To make such a pad 1, there is provided a method according to which a supply of liquid- and gas-pervious cover sheet 4 of indeterminate length is fed to the periphery of a multi-perforate drum whose holes match the size of the bodies to be formed. Bodies are formed in the cover sheet 4 material, these bodies being disposed one in each hole in the drum. If the cover sheet 4 is thermo-formable, as in the case of a thermoplastic resin, the bodies 3 can be formed by drawing a vacuum on the radially inner side of the drum while supplying heated air from the radially outer side thereof, so that the heated air passes through the thermo-formable porous material and softens the same, the resistance to this passage exerting sufficient force on the material to form the bodies 3. In the case of a non-thermo-formable material, the bodies 3 can be formed by mechanical embossing, or by creasing the material both lenghwise and transversely so as to provide an excess of material in both directions, and then drawing this excess material down into pockets by suction.

As the drum turns, the pre-formed pockets will move into a body filling station in which absorbent material such as a fluff of bleached sulphate pulp is drawn into the pockets, again by vacuum from the inner side of the drum. As the vacuum draws the fluff into the pockets, the material of the pockets serves as a filter, letting the air pass through toward the source of vacuum but retaining the absorbent material in the pockets. The surfaces of the cover sheet 4 that overlie the bridges between the holes through the drum, however, are not subject to vacuum and so do not tend to accumulate absorbent material thereon.

A liquid-impervious back sheet 2 is then applied and is secured directly to those portions of the cover sheet 4 that overlie the bridges between the holes through the drum. This can be done by applying glue between the back sheet 2 and the bridge portions of the cover sheet, or by heat welding. The final product is finally stripped from the drum and cut to size. This method of absorbent fluff supply is entirely conventional and as such forms no part of the present invention.

The supply of absorbent material to a chamber, and the dwell time of the pockets therein, as well as the setting of fans, are such that the bodies are just full but not overfilled with absorbent material. The absorbent material is strongly drawn into the pockets and held there by the air flow induced by the vacuum in the chamber, with the result that substantially no absorbent material is left on that portion of the cover sheet 4 that overlies the bridges between the holes of the drum.

Of course, it is also possible to provide a brush or other screed (not shown) to sweep free from any possible absorbent material, the exposed portions of the cover sheet 4 between the bodies 3. Turning further, the drum brings the filled pockets to a glue spray for securing the liquid-impervious back sheet 2.

The glue for this purpose is preferably a hot melt glue of conventional type for use in disposable diapers and the like. The liquid-impervious back sheet 2 is fed from a supply thereof about rollers of which a roller presses the back sheet 2 against those exposed portions of the cover sheet 4 that are backed by the bridges between the holes of the drum.

In the illustrated embodiment, the glue has been sprayed against those bridge backed exposed portions of the cover sheet 4. Alternatively, of course, the glue spray can be directed against the impervious sheet 2 itself, for example as it passes about the roller. In any event, the rate of spray application of the melt adhesive is 1-20 g/m, preferably 3-5 g/m. Still another alternative is to dispense with the glue spray altogether and to bond the sheets 2, 4 and together along the locations of the channels 7, by heat welding. In this case, a roll will be heated to perform a conventional heat sealing operation. To facilitate this operation, a coextruded film (not shown) can be used-for the base sheet, having a thin layer of easily heat-sealable material on the side thereof that will contact the cover sheet.

The finished product finally leaves the periphery of the drum, eventually to be cut to the desired size of pads 1 by conventional cutting means (not shown), or can be directly cut to size without rolling (not shown).

Whether the material of the cover sheet 4 is or is not thermoplastic, it can be mechanically preformed to the shape of the required pockets by passing it between embossing rolls or belts or the like, it being possible to use a drum itself as one of the embossing rolls.

Although the present invention has been described and illustrated in connection with preferred embodiments, it is to be understood that modifications and variations may be resorted to without departing from the spirit of the invention, as those skilled in this art will readily understand. Such modifications and variations are considered to be within the purview and scope of the present invention as defined by the appended claims.

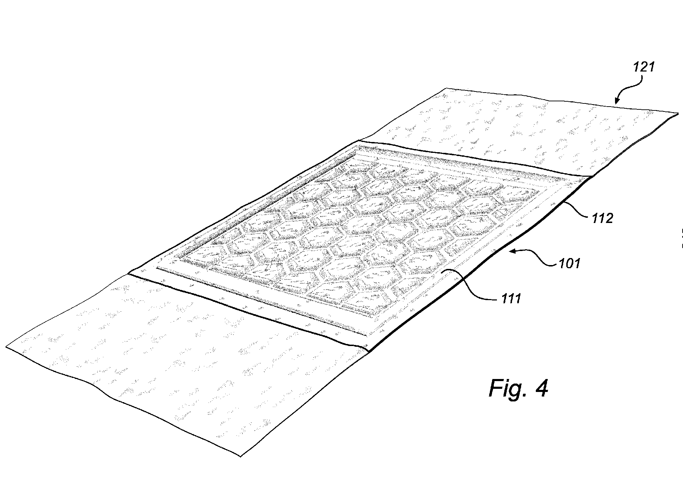

According to a second embodiment as disclosed in fig. 4 the absorbent pad may be provided with wings 121 in order to facilitate applying and securing the same in a bed. The other features are as descrided before. Preferably the wings 121 are made of a non-woven material that is attached to one side of the absorbent pad and another wing 121 is attached to an opposite side of the absorbent pad.

Although it is preferred that the channels 7 define between them islands of absorbent material, that is, bodies 3 of absorbent material bounded on all sides by the channels 7, it is also within the scope of the invention that at least some of the bodies of absorbent material be more or less continuous. For example, it is within the invention to arrange at least some of the channels in the form of an advertising symbol or logo.

Advantageously, the bodies are hexagonal and the joints thus splitted in 3 channels with 120 degrees in between as seen in the plane of the pad.

## Claims

1. An absorbent pad (1) comprising a liquid-impervious back sheet (2), a plurality of bodies (3) of liquid-absorbent material disposed on one surface of the back sheet (2), and a liquid-pervious cover sheet (4) covering said bodies of absorbent material and secured to the back sheet (2) along channels (7) which separate said bodies (3) of absorbent material,
**characterized in that**
the channels (7) progresses in a channel-pattern in which three channels (7), meeting in a joint, form respective edges (6) of said whole bodies (3) and said channels (7) being substantially free from absorbent material.

2. An absorbent pad (1) as claimed in claim 1, in which said edges (6) of said whole body (3) has an angle (α) of 105-135°, preferably 118-122°, in relation to nearlying edges (6) thereof.

3. An absorbent pad (1) as claimed in any one of claims 1-2, in which said cover sheet (4) is adhesively bonded to said back sheet (2) propagating along said channels (7).

4. An absorbent pad (1) as claimed in any one of claims 1-3, in which said cover sheet (4) is cohesively secured to said back sheet (2) along said channels (7).

5. An absorbent pad (1) as claimed in any one of claims 1-4, in which said back sheet (2) is substantially flat and said cover sheet (4) has pockets therein and containing said bodies (3).

6. An absorbent pad (1) as claimed in any one of claims 1-5, in which said bodies (3) contains 6 edges (6).

7. An absorbent pad (1) as claimed in any one of claims 1-5, in which said three channels (7) propagates evenly distributed, in the plane of said absorbent pad (1), from said joint.

8. A method of making an absorbent pad (1) from a liquid-impervious back sheet (2) and a liquid-pervious cover sheet (4) and absorbent material between said sheets, comprising forming in the cover sheet a plurality of bodies (3), filling the bodies (3) with a separate quantity of absorbent material in each body (3), and securing the back sheet (2) to the cover sheet along channels between the bodies (3),
**characterized in that**
the channels (7) progresses in a channel-pattern in which three channels (7) meet in joints forming respective edges (6) of said whole bodies (3) and said channels (7) being substantially free from absorbent material.
